Europäisches Patentamt

European Patent Office

Office européen des brevets

(11) Veröffentlichungsnummer: **0 396 074**
**A1**

## (12) EUROPÄISCHE PATENTANMELDUNG

(21) Anmeldenummer: **90108191.9**

(22) Anmeldetag: **28.04.90**

(51) Int. Cl.5: **A61M 29/00**

(30) Priorität: **03.05.89 CH 1687/89**

(43) Veröffentlichungstag der Anmeldung:
**07.11.90 Patentblatt 90/45**

(84) Benannte Vertragsstaaten:
**BE DE FR IT NL**

(71) Anmelder: **Sterimed Gesellschaft für medizinischen Bedarf mbH**
**Fasanerieweg 13-17 Postfach 215**
**D-6600 Saarbrücken 3(DE)**

(72) Erfinder: **Kramann, Bernard**
**Preussenstrasse 43**
**D-6650 Homburg/Saar(DE)**

(74) Vertreter: **RUPP, Herbert Byk Gulden Lomberg**
**Chemische Fabrik GmbH et al**
**Byk-Gulden-Strasse 2 Postfach 6500**
**D-7750 Konstanz(DE)**

(54) **Vorrichtung für die Dilatation von Gefässen.**

(57) Eine Vorrichtung, umfassend eine drahtförmige elastische Sonde (3) mit einer olivenförmigen Verdickung (5) im Bereich des distalen Endes (6) in einem Katheterschlauch (4), dadurch gekennzeichnet, daß die Sonde eine Länge von 150 bis 200 cm und einen Durchmesser von 0,5 bis 1,5 mm hat und die olivenförmige Verdickung 5 einen Durchmesser von 1,5 bis 3 mm hat und die Sonde 3 einschließlich olivenförmiger Verdickung 5 eine Hydrogel-Beschichtung aufweist, eignet sich zur Dilatation peripherer Gefäße.

Fig.1

EP 0 396 074 A1

## Vorrichtung für die Dilatation von Gefäßen

### Technisches Gebiet

Die Erfindung bezieht sich auf eine Vorrichtung für die Dilatation von peripheren Gefäßen, umfassend eine drahtförmige elastische Sonde mit einer olivenförmigen Verdickung im Bereich des distalen Endes in einem Katheterschlauch.

### Stand der Technik

Bei Verengung von Gefäßen, beispielsweise durch arteriosklerotische Prozesse in Herzkranzgefäßen, ist es seit langem üblich, durch Einbringen von Kathetern mit aufblasbaren Ballonen eine Dilatation zu bewerkstelligen. Diese Methode hat die bereits vorher üblich gewesene Dilatation mit Hilfe eines Satzes teleskopartig koaxial ineinander geschobener Katheter weitgehend abgelöst. Durch die Entwicklung immer dünnerer Katheter und Ballone ist es möglich geworden, auch periphere Gefäße zu erreichen und zu weiten. Durch die Verwendung von oberflächlich mit Hydrogelen beschichteten Materialien konnten Haft- und Gleitreibung an den Gefäßoberflächen stark gesenkt werden.

In neuerer Zeit ist der Wunsch aufgetaucht, auch sehr periphere Gefäße, beispielsweise im Bereich des Unterschenkels, zu dilatieren. Bei solchen Anwendungen ist es oft nicht möglich, die Ballone durch die stark verengten Gefäße hindurchzuzwängen, um sie anschließend zum Zwecke der Dilatation aufzublasen.

### Beschreibung der Erfindung

Aufgabe der vorliegenden Erfindung ist es, eine Vorrichtung für die Dilatation von Gefäßen zur Verfügung zu stellen, die vor allem für Gefäße anwendbar ist, für die wegen ihres geringen Lumens die Ballonmethode nicht anwendbar ist.

Gelöst wird diese Aufgabe dadurch, daß die Sonde eine Länge von 150 bis 200 cm und einen Durchmesser von 0,5 bis 1,5 mm hat und die olivenförmige Verdickung einen Durchmesser von 1,5 bis 3 mm hat und die Sonde einschließlich olivenförmiger Verdickung eine Hydrogel-Beschichtung aufweist. Vorteilhaft weist auch der Katheterschlauch eine Hydrogel-Beschichtung auf. Gegenstand der Erfindung sind die in den Ansprüchen definierten Gegenstände.

Sonde und Katheterschlauch bestehen aus den für solche Gegenstände auf dem Gebiet von Angioplastiekathetern üblichen Materialien und werden nach den gängigen Herstellungsmethoden hergestellt. Das distale Ende der Sonde ist, wie bei Führungsdrähten für Angiographiekatheter üblich, als flexible Spitze gestaltet. Zwischen dieser flexiblen Spitze und dem steiferen Stamm der Sonde befindet sich die olivenartige Verdickung. Die Sonde einschließlich der olivenartigen Verdickung weist eine hydrophile Oberfläche auf.

Die Erzeugung von hydrophilen Oberflächen, die durch Wasseraufnahme ein Hydrogel ausbilden, ist bekannt. In GB-A-2131442 werden hydrophile Polyurethan/Polyen-Zusammensetzungen beschrieben. Hydrophile Polyurethan/Diacrylat-Zusammensetzungen sind aus GB-A-2086927 bekannt. Die Herstellung medizinischer Artikel mit hydrophilen Oberflächen aus einem Methacrylsäure/2-Hydroxyethylmethacrylat-Copolymer ergibt sich aus US-A-4527293.

Je nach Anwendungszweck weist die Sonde eine Länge von 150 bis 200 cm, vorzugsweise 150 bis 160 cm auf. Der Durchmesser der Sonde beträgt 0,5 bis 1,5 mm, vorzugsweise 0,8 bis 1,3 mm. Die olivenförmige Verdickung im Bereich des distalen Endes der Sonde weist einen Durchmesser von 1,5 bis 3 mm, vorzugsweise 2 mm bis 3 mm auf.

Die Anwendung der erfindungsgemäßen Vorrichtung wird in Anlehnung an die bei der Dilatation von Gefäßen geübten Praktiken durchgeführt. Beispielsweise wird zur Dilatation peripherer Gefäße im Unterschenkel in die Leistenbeuge eine Schleuse gelegt. Bevor die erfindungsgemäße Vorrichtung durch die Schleuse in das Gefäß eingeführt wird, kann gewünschtenfalls ein Röntgenkontrastmittel in das Gefäß eingespritzt werden. Sonde und Katheterschlauch werden - zweckmäßigerweise unter Durchleuchtungskontrolle - bis zu der zu dilatierenden Gefäßverengung vorgeschoben. Der Katheterschlauch hat hierbei im wesentlichen die Aufgabe, die Sonde zu stabilisieren. Durch Hineinschieben der olivenförmigen Verdickung der Sonde in den verengten Bereich des Gefäßes wird sodann eine Dilatation bewirkt. Gewünschtenfalls kann nach Entfernen der erfindungsgemäßen Vorrichtung eine weitere Dilatation des Gefäßes durch einen Dilatationskatheter nach dem Stand der Technik vorgenommen werden.

Es zeigt sich, daß durch die baulich sehr einfache erfindungsgemäße Vorrichtung der Anwendungsbereich der Gefäßdilatation weit in Richtung auf sehr enge periphere Gefäße ausgedehnt werden kann.

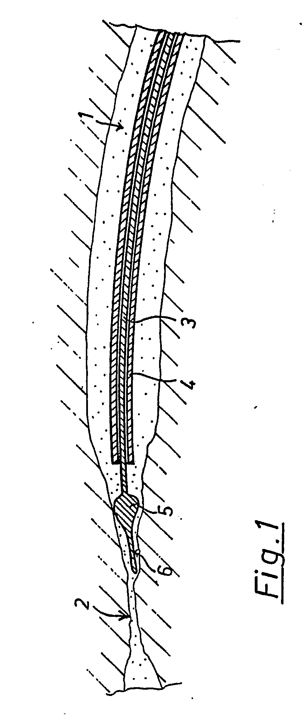

Nachstehend soll die Erfindung anhand der Fig. 1, die schematisch einen Längsschnitt durch eine Sonde mit Katheterschlauch in einer verengten Arterie zeigt, erläutert werden.

Das flexible distale Ende 6 der drahtförmigen Sonde 3 wird zusammen mit Katheterschlauch 4 bis zur Verengung 2 einer Arterie 1 vorgeschoben. Zwischen dem flexiblen distalen Ende 6 und dem steiferen Stamm der Sonde 3 befindet sich die olivenförmige Verdickung 5. Durch weiteres Vorschieben der Sonde 3 in die Verengung 2 der Arterie 1 erfolgt eine Dilatation.

**Ansprüche**

1. Vorrichtung für die Dilatation von peripheren Gefäßen, umfassend eine drahtförmige elastische Sonde mit einer olivenförmigen Verdickung im Bereich des distalen Endes in einem Katheterschlauch, dadurch gekennzeichnet, daß die Sonde (3) eine Länge von 150 bis 200 cm und einen Durchmesser von 0,5 bis 1,5 mm hat und die olivenförmige Verdickung (5) einen Durchmesser von 1,5 bis 3 mm hat und die Sonde (3) einschließlich olivenförmiger Verdickung (5) eine Hydrogel-Beschichtung aufweist.

2. Vorrichtung nach Anspruch 1, dadurch gekennzeichnet, daß der Katheterschlauch (4) eine Hydrogel-Beschichtung aufweist.

*Fig.1*

EP 0 396 074 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | US-A-1 878 671 (CANTOR) <br> * Figuren 1,2; Anspruch 1 * <br> --- | 1-2 | A 61 M 29/00 |
| Y | WO-A-8 801 885 (VERSAFLEX DELIVERY SYSTEMS INC.) <br> * Seite 21, Zeile 13 - Seite 22, Zeile 7 * <br> --- | 1-2 | |
| A | US-A-3 196 876 (MILLER) <br> * Figur 4 * <br> --- | 1 | |
| A | US-A-3 867 329 (HALPERN et al.) <br> * Spalte 2, Zeilen 15-42 * <br> --- | 1-2 | |
| A | GB-A-2 179 258 (MILLER) <br> * Zusammenfassung; Seite 1, Zeilen 21-25 * <br> ----- | 1-2 | **RECHERCHIERTE SACHGEBIETE (Int. Cl.5)** <br><br> A 61 M |

Der vorliegende Recherchenbericht wurde für alle Patentansprüche erstellt

| Recherchenort | Abschlußdatum der Recherche | Prüfer |
|---|---|---|
| DEN HAAG | 02-08-1990 | MIR Y GUILLEN V. |

EPO FORM 1503 03.82 (P0403)